# EUROPÄISCHE PATENTANMELDUNG

(11) **EP 1 430 880 A2**
(43) Veröffentlichungstag der Anmeldung: **23.06.2004**
(21) Anmeldenummer: 03028618.1
(22) Anmeldetag: 11.12.2003
(51) Int. Cl.: A61K 7/32

(54) **Alkoholfreie Deosprays mit hautkühlenden Wirkstoffen**

(30) Priorität: 20.12.2002 DE 10260957
(71) Anmelder: Hans Schwarzkopf & Henkel GmbH & Co. KG, 22763 Hamburg (DE)
(72) Erfinder: Emmerling, Winfried, Dr., 25436 Tornesch (DE); Heinsohn, Ulrike, 22529 Hamburg (DE)
(74) Vertreter: Strohe-Kamp, Geertje

(57) **Zusammenfassung**

Gegenstand der vorliegenden Erfindung sind alkoholfreie Deosprays, die nicht-verkapselte Parfumöle, verkapselte Parfumöle und verkapselte hautkühlende Wirkstoffe enthalten und nach der Anwendung einen verzögerten Frischeeffekt hervorrufen.

## Beschreibung

Gegenstand der vorliegenden Erfindung sind alkoholfreie Deosprays, die nicht-verkapselte Parfumöle, verkapselte Parfumöle und verkapselte hautkühlende Wirkstoffe enthalten und nach der Anwendung einen verzögerten Frischeeffekt hervorrufen.

Deosprays enthalten neben antimikrobiellen und/oder schweißreduzierenden Wirkstoffen in der Regel eine variantenspezifische Parfümierung. Die erfrischende bzw. geruchsüberdeckende Funktion des Parfums ist für die Gesamtbeurteilung der Leistungsfähigkeit eines Deosprays mindestens genau so wichtig wie die deodorierenden Komponenten der Formulierung. Diese Frischewirkung ist kurz nach der Applikation des Deosprays natürlich am ausgeprägtesten, nimmt aber mit der Zeit durch Verdunsten des Parfums ab. Parallel dazu empfinden viele Verbraucher, dass die Leistungsfähigkeit der Gesamtformulierung mit abnehmender Intensität des Parfums ebenfalls abnimmt.

Bei den handelsüblichen Deosprays wird ein Großteil der erfrischenden Wirkung durch einen Gehalt an flüchtigen Lösemitteln, insbesondere an Ethanol oder flüchtigen Siliconölen wie Cyclomethicone, erzielt. Ein hoher Ethanolgehalt kann allerdings auf der besonders empfindlichen Haut der Achselhöhlen zu Überempfindlichkeitsreaktionen führen. Ein hoher Gehalt an Cyclomethicone oder anderen Siliconölen ist aus Kostengründen nachteilig.

Es gab daher Versuche, Deosprays mit verkapselten Parfumölen zu entwickeln, die sich unter Feuchtigkeitseinfluss, d. h. beim Schwitzen oder unter längerem Einfluss der normalen Hautfeuchtigkeit, öffnen und das verkapselte Parfum freisetzen. In der Druckschrift EP 279 328 A2 ist ein System beschrieben, das das gleiche Parfum sowohl in verkapselter als auch in freier Form enthält. In der Druckschrift DE 4403913 A1 ist ein System beschrieben, bei dem sich das verkapselte Parfum von dem in der Formulierung frei vorliegenden Parfum unterscheidet. Hierdurch kann ein zusätzlicher Dufteffekt besser wahrgenommen werden als bei dem System der EP 279 328 A2. Dennoch erscheint dieser Frische-"Boost" verbesserungsbedürftig.

Überraschend wurde nun gefunden, dass die Freisetzung eines Parfums, insbesondere eines Parfums mit einer Frischenote, aus einer wasserlöslichen Verkapselung verstärkt wird, wenn sie durch einen hautkühlenden Wirkstoff unterstützt wird. Dabei erzielen die erfindungsgemäßen Deosprays auch ohne Ethanol einen hohen und gleichzeitig angenehmen Frischeeffekt.

Gegenstand der vorliegenden Erfindung sind daher alkoholfreie Deosprays, die mindestens ein nicht-verkapseltes Parfumöl bzw. eine nicht-verkapselte Parfumöl-Mischung, mindestens ein verkapseltes Parfumöl bzw. eine verkapselte Parfumöl-Mischung, mindestens einen verkapselten hautkühlenden Wirkstoff und mindestens ein Treibgas enthalten, wobei das verkapselte Parfumöl und der hautkühlende Wirkstoff mit mindestens einem wasserlöslichen Polymer verkapselt sind.

Unter Feuchtigkeitseinfluss öffnet sich eine gewisse Zeit nach der Applikation das wasserlösliche Kapselmaterial, und das verkapselte Parfumöl sowie der verkapselte hautkühlende Wirkstoff werden erst nach eine gewisse Zeit nach und nicht größtenteils schon während der Applikation freigesetzt.
Verkapselte und nicht-verkapselte Parfumöle bzw. Parfumöl-Mischungen können gleich oder verschieden sein. In einer bevorzugten Ausführungsform der Erfindung sind die verkapselten und die nicht-verkapselten Parfumöle bzw. Parfumöl-Mischungen voneinander verschieden.
Der Gehalt an nicht-verkapseltem Parfumöl beträgt erfindungsgemäß 0,1 - 2 Gew.%, bevorzugt 0,2 - 1,5 Gew.% und besonders bevorzugt 0,4 - 1 Gew.%, jeweils bezogen auf das Gesamtgewicht der Formulierung.
Der Gehalt an verkapselten Parfumöl beträgt 0,01 - 2 Gew.%, bevorzugt 0,1 - 1,0 Gew.% und besonders bevorzugt 0,25 - 0,5 Gew.%, jeweils bezogen auf das Gesamtgewicht der Formulierung.

Das verkapselte Parfumöl und der verkapselte hautkühlende Wirkstoff können gemeinsam als Mischung verkapselt vorliegen. Es können aber auch beide Komponenten getrennt nebeneinander verkapselt sein. In einer bevorzugten Ausführungsform der Erfindung sind Parfumöl und hautkühlender Wirkstoff gemeinsam als Mischung verkapselt.
Das Gewichtsverhältnis von verkapseltem Parfumöl und verkapseltem hautkühlenden Wirkstoff beträgt erfindungsgemäß von 10:1 bis 1:10.

Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Zu den phenolischen Riechstoffverbindungen zählt z. B. Carvacrol. Riechstoffverbindungen vom Typ der Ester sind z.B. Benzylacetat, Methylanthranilat, ortho-t-Butylcyclohexylacetat, p-tert.-Butylcyclohexylacetat, Diethylphthalat, Nonandiol-1,3-diacetat, iso-Nonylacetat, iso-Nonylformiat, Phenylethylphenylacetat, Phenoxyethylisobutyrat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Benzylsalicylat, Ethylsalicylat, iso-Amylsalicylat, Hexylsalicylat und 4-Nonanolid. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. 6-Acetyl-1,1,3,4,4,6-hexamethyltetrahydronaphthalin, para-t-Amylcyclohexanon, 2-n-Heptylcyclopentanon, β-Methylnaphthylketon und die lonone α-Isomethylionon und Methylcedrylketon, zu den Alkoholen Zimtalkohol, Anethol, Citronellol, Dimyrcetol, Eugenol, Geraniol, Linalool, Phenylethylalkohol und Terpineol, zu den Kohlenwasserstoffen gehören 1,3,4,6,7,8-Hexahydro-4,6,6,7,8,8-hexamethylcyclopenta-a-2-benzopyran, Hydroxymethylisopropylcyclopentan, 3-a-Methyldodecahydro-6,6,9a-trimethylnaphtho-2(2,1-b)furan, iso-Butylchinolin sowie die Terpene und Balsame. Bevorzugt werden Mischungen verschiedener Riechstoffe verwendet, die gemeinsam eine ansprechende Duftnote erzeugen.
Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, isoEugenol, Thymianöl, Bergamotteöl, Geraniumöl und Rosenöl.

Erfindungsgemäß geeignete hautkühlende Wirkstoffe sind beispielsweise Menthol, Iso-pulegol sowie Mentholderivate, z. B. Menthyllactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol. Als hautkühlende Wirkstoffe bevorzugt sind Menthol, Isopulegol, Menthyllactat, Menthoxypropandiol und Menthylpyrrolidoncarbonsäure.
Die erfindungsgemäßen Formulierungen enthalten den hautkühlenden Wirkstoff in Mengen von 0,01 - 1 Gew.%, bevorzugt 0,02 - 0,5 Gew.% und besonders bevorzugt 0,05 - 0,2 Gew.%, jeweils bezogen auf das Gesamtgewicht der Formulierung.

Als Kapselmaterial geeignet sind wasserlösliche Polymere wie Stärke, physikalisch und/oder chemisch modifizierte Stärken, Cellulosederivate, wie z. B. Carboxymethylcellulose, Methylcellulose, Hydroxyethylcellulose oder Hydroxypropylmethylcellulose, Carragheene, Alginate, Maltodextrine, Dextrine, Pflanzengummen, Pektine, Xanthane, Polyvinylacetat und Polyvinylalkohol, Polyvinylpyrrolidin, Polyamide, Polyester und Homo- und Copolymere aus Monomeren, ausgewählt aus Acrylsäure, Methacrylsäure, Maleinsäure, Fumarsäure, Itaconsäure sowie den Estern und den Salzen dieser Säuren, sowie beliebige Mischungen dieser Polymeren.

Bevorzugte Kapselmaterialien sind chemisch modifizierte Stärken, insbesondere Aluminiumstärkeoctenylsuccinat, z. B. das Handelsprodukt Dry Flo Plus von National Starch, oder Natriumstärkeoctenylsuccinat, z. B. das Handelsprodukt Tylose H 10 von Clariant, desweiteren die Carboxymethylcellulose, Carboxymethylcellulose, Methylcellulose, Hydroxyethylcellulose und Hydroxypropylmethylcellulose, weiterhin Carragheene, Alginate und Maltodextrine, sowie beliebige Mischungen dieser Polymere.

Besonders bevorzugte Kapselmaterialien sind Polymermischungen, die aus chemisch modifizierten Stärken und/oder Hydroxyethylcellulose und einem Anteil von 0,2 - 2 Gew.% an Alginaten und/oder Carragheenen bestehen.

Die Verkapselung kann nach bekannten Verfahren erfolgen. Entsprechende Verfahren sind z. B. offenbart in K. Master, "Spray Drying Handbook", 3. Auflage, John Wiley, 1979. In einem besonders bevorzugten Verkapselungsverfahren wird eine Mischung auf Wasserbasis hergestellt, die etwa 20 - 50 Gew.% des polymeren Verkapselungsmaterials, etwa 0,1 - 2,0 Gew.% eines Emulgators, etwa 5 - 20 Gew.% des zu verkapselten Parfumöls und/oder des zu verkapselten hautkühlenden Wirkstoffs sowie etwa 40 - 60 Gew.% Wasser enthält. Diese Mischung wird homogenisiert und anschließend sprühgetrocknet. Die wirkstoffbeladenen Kapseln werden so als feines Pulver mit einem Teilchendurchmesser von 1 - 150 µm, bevorzugt 20 - 80 µm, besonders bevorzugt 5 - 50 µm, erhalten.
In einem anderen Herstellverfahren erfolgt die Mikroverkapselung durch Koazervation, wobei der Träger bevorzugt aus Gelatine gebildet wird.

Das Kapselmaterial, bestehend aus wasserlöslichen Polymeren und einem geringen Gehalt an Emulgatoren, ermöglicht eine reversible "Wiederverkapselung" der verkapselten Parfumöle und hautkühlenden Wirkstoffe. Die Wiederverkapselung tritt dabei in situ während des Trocknens der Haut, das einer Perspirationsperiode folgt, auf. So treten verschiedene, aufeinander folgende Aktivierungen auf der Haut ein, ohne dass der Benutzer eine weitere Anwendung des erfindungsgemäßen Mittels vornehmen muss.

Als Emulgatoren sind nicht-ionische Emulgatoren, und hierbei insbesondere die hydrophilen nicht-ionischen Emulgatoren, d. h. solche mit einem HLB-Wert > 8, geeignet. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert erfindungsgemäß nach folgender Formel berechnet: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist. Der O/W-Emulgator wird in einer Menge von 0,1 - 10 Gew.-%, vorzugsweise 0,1 - 5 Gew.-% und insbesondere 0,5 - 3 Gew.-% bezogen auf die Gesamtzusammensetzung eingesetzt. Auch der Einsatz eines Gemisches von O/W-Emulgatoren kann vorteilhaft sein.
Um besonders feinteilige Dispersionen zu erhalten, ist es vorteilhaft, eine Kombination von nichtionischen Emulgatoren einzusetzen. Zu den geeigneten nichtionischen Emulgatoren gehören beispielsweise:
(1) Anlagerungsprodukte von 2 bis 50 Mol Ethylenoxid und/oder 0 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe;
(2) Phosphorsäuretriester von C₆ - C₂₂-Alkoholen sowie deren Ethylenoxidanlagerungsprodukte;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga.
(5) Anlagerungsprodukte von 15 bis 60 Mol Ethylenoxid an Ricinusöl und/oder gehärtetes Ricinusöl;
(6) C_{12/18}-Fettsäuremono- und -diester von Anlagerungsprodukten von 1 bis 50 Mol Ethylenoxid an Glycerin;
(7) Polyol- und insbesondere Polyglycerinester, wie z. B. Polyglycerinpolyricinoleat, Polyglycerinpoly-12-hydroxystearat oder Polyglycerindimerat; ebenfalls geeignet sind Gemische von Verbindungen aus mehreren dieser Substanzklassen;
(8) Polyalkylenglykole;
(9) Mischester aus Pentaerythrit, Fettsäuren, Citronensäure und Fettalkohol gemäß **DE 1165574** und/oder Mischester von Fettsäuren mit 6 bis 22 Kohlenstoffatomen, Methylglucose und Polyolen, vorzugsweise Glycerin oder Polyglycerin.

In einer bevorzugten Ausführungsform enthält die Zusammensetzung mindestens einen nichtionischen Emulgator mit einem HLB-Wert von 8 - 18.

Besonders bevorzugte nicht-ionische Emulgatoren mit einem HLB-Wert > 8 sind Glycerinmono- und Difettsäureester, die Fettsäureester von Sorbitol und von Mono- und Disacchariden, insbesondere von Sucrose sowie deren alkoxylierten Derivaten, sowie die Phosphorsäuretriester von ethoxylierten C₆ - C₂₂ - Alkoholen.

Der Begriff "alkoholfrei" wird erfindungsgemäß so verstanden, dass die Zusammensetzungen frei ist von ein- oder zweiwertigen C₁ - C₆-Alkoholen, die keine Duftalkohole sind, enthalten. In einer besonders bevorzugten Ausführungsform sind die erfindungsgemäßen Zusammensetzungen frei von Ethanol oder Isopropanol.

Die erfindungsgemäßen Zusammensetzungen sind im wesentlichen wasserfrei, d. h. sie enthalten 0 bis maximal 2 Gew.% Wasser.

Die erfindungsgemäß bevorzugten Treibgase sind ausgewählt aus Propan, Butan, Isobutan, Pentan, Isopentan, Dimethylether, Kohlendioxid, Distickstoffoxid, Fluorkohlenwasserstoffen und Fluorchlorkohlenwasserstoffen sowie Mischungen dieser Substanzen.

Die Treibgase sind in Mengen von 20 - 95 Gew.%, bevorzugt 30 - 95 Gew.% und besonders bevorzugt 60 - 95 Gew.%, jeweils bezogen auf die gesamte Aerosolzusammensetzung, enthalten.

Weiterhin können die erfindungsgemäßen Zusammensetzungen zusätzliche Deodorantien enthalten. Als Deodorantien können antimikrobielle, antibakterielle oder keimhemmende Stoffe, Antioxidantien oder Geruchsadsorbentien (z. B. Zinkricinoleat) eingesetzt werden.
Geeignete antimikrobielle, antibakterielle oder keimhemmende Stoffe sind insbesondere Organohalogenverbindungen sowie -halogenide, quartäre Ammoniumverbindungen, eine Reihe von Pflanzenextrakten und Zinkverbindungen. Bevorzugt sind halogenierte Phenolderivate wie z. B. Hexachlorophen oder Irgasan DP 300 (Triclosan, 2,4,4'-Trichlor-2'-hydroxydiphenylether), 3,4,4'-Trichlorcarbonilid, Chlorhexidin (1,1'-Hexamethylen-bis-[5-(4-chlorphenyl)]-biguanid), Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar. Auch schwächer wirksame antimikrobielle Stoffe, die aber eine spezifische Wirkung gegen die für die Schweißzersetzung verantwortlichen grampositiven Keime haben, können als Deodorant-Wirkstoffe eingesetzt werden. Auch Benzylalkohol, die Ester von aliphatischen C₂-C₆-Carbonsäuren oder Hydroxycarbonsäuren und C₂-C₆-Alkoholen oder Polyolen, z. B. Triethylcitrat, Propylenglycollactat, Polyglycerincaprylat oder Glycerintriacetat (Triacetin), können als Deodorant-Wirkstoff eingesetzt werden. Weitere antibakteriell wirksame Deodorantien sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide. Ebenfalls geeignet sind langkettige Diole, z. B. 1,2-Alkan-(C₈-C₁₈)-Diole, Glycerinmono-(C₆-C₁₆)-alkylether oder Glycerinmono(C₈-C₁₈)-Fettsäureester, die sehr gut haut- und schleimhautverträglich und gegen Corynebakterien wirksam sind.
Antioxidative Stoffe können der oxidativen Zersetzung der Schweißkomponenten entgegenwirken und auf diese Weise die Geruchsentwicklung hemmen. Geeignete Antioxidantien sind Imidazol und Imidazolderivate (z. B. Urocaninsäure), Peptide wie z. B. D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis µmol/kg), ferner Metallchelatoren (z. B. α-Hydroxyfettsäuren, EDTA, EGTA, Lactoferrin), Huminsäuren, Gallensäure, Gallenextrakte, Catechine, Bilirubin, Biliverdin und deren Derivate, ungesättigte Fettsäuren und deren Derivate (z. B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, -dipalmitat, -acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate, Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Selen und Selen-Derivate (z. B. Selenmethionin), Stilbene und Stilben-Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte, die diese Antioxidantien enthalten, eingesetzt werden.
Als lipophile, öllösliche Antioxidantien aus dieser Gruppe sind Tocopherol und dessen Derivate, insbesondere Tocopherylacetat, und Carotinoide sowie Butylhydroxytoluol/anisol bevorzugt. Als wasserlösliche Antioxidantien sind Aminosäuren, z. B. Tyrosin und Cystein und deren Derivate sowie Gerbstoffe, insbesondere solche pflanzlichen Ursprungs, bevorzugt.

Die Gesamtmenge der Antioxidantien in den erfindungsgemäßen Zubereitungen beträgt 0,001 - 10 Gew.-%, vorzugsweise 0,05 - 5 Gew.-% und insbesondere 0,1 - 2 Gew.-%, bezogen auf die gesamte Zubereitung.
Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind z. B. die Salze der Ethylendiamintetraessigsäure oder der Nitrilotriessigsäure sowie die Salze der 1-Hydroxyethan-1,1-diphosphonsäure.

Die erfindungsgemäßen Zusammensetzungen enthalten in einer bevorzugten Ausführungsform mindestens einen Antitranspirant-Wirkstoff. Als Antitranspirant-Wirkstoffe eignen sich wasserlösliche adstringierende metallische Salze, insbesondere anorganische und organische Salze des Aluminiums, Zirkoniums und Zinks bzw. beliebige Mischungen dieser Salze. Erfindungsgemäß wird unter Wasserlöslichkeit eine Löslichkeit von wenigstens 5 g Aktivsubstanz pro 100 g Lösung bei 20 °C verstanden. Erfindungsgemäß verwendbar sind beispielsweise Alaun (KAl(SO₄)₂ · 12 H₂O), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat und Aluminium-Zirkonium-Chlorohydrat-Glycin-Komplexe. Bevorzugt enthalten die Zusammensetzungen ein adstringierendes Aluminiumsalz, insbesondere Aluminiumchlorohydrat, und/oder eine Aluminium-Zirkonium-Verbindung. In den erfindungsgemäßen wasserfreien Zusammensetzungen werden die Antitranspirant-Wirkstoffe in fester Form eingesetzt. Sie sind in den erfindungsgemäß in einer Menge von 1 - 40 Gew.-%, vorzugsweise 5 - 30 Gew.-% und insbesondere 10 - 25 Gew.-% enthalten (bezogen auf die Menge der Aktivsubstanz in der Gesamtzusammensetzung). Aluminiumchlorohydrate werden beispielsweise pulverförmig als Micro Dry® Ultrafine von Reheis, als Chlorhydrol®, sowie in aktivierter Form als Reach® 501 oder Reach® 103 von Reheis vertrieben. Unter der Bezeichnung Reach® 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal® 36G Powder im Handel sind, ist erfindungsgemäß besonders vorteilhaft.

Die erfindungsgemäßen Zusammensetzungen enthalten in einer bevorzugten Ausführungsform mindestens ein Suspensions- oder Verdickungsmittel. Besonders geeignete Verdickungsmittel sind hydrophobierte Tonmineralien wie Montmorillonite, Hectorite und Bentonite, insbesondere Disteardimonium Hectorite und Quaternium-18 Hectorite. Die handelsüblichen Verdickungsmittel stellen diese hydrophobierten Tonmineralien in Form eines Gels in Cyclomethicone und gewünschtenfalls einer zusätzlichen Ölkomponente, wie z. B. Propylencarbonat, bereit. Weitere geeignete Verdickungsmittel sind pyrogene Kieselsäuren, z. B. die Handelsprodukte der Aerosil®-Serie von Degussa.

Die erfindungsgemäßen Zusammensetzungen können in handelsüblichen Aerosoldosen verpackt sein. Die Dosen können aus Weißblech oder aus Aluminium sein. Weiterhin können die Dosen innen beschichtet sein, um die Gefahr der Korrosion so gering wie möglich zu halten.
Die Dosen sind mit einem geeigneten Sprühkopf ausgestattet. Je nach Sprühkopf sind Ausstoßraten, bezogen auf voll gefüllte Dosen, von 0,1 g/s bis 2,0 g/s möglich.

Die nachfolgenden Beispiele sollen die Erfindung verdeutlichen, ohne sie hierauf zu beschränken.

### Wirkungsnachweis:

In einem Anwendungstest wurde die verbesserte Produktleistung der erfindungsgemäßen Zusammensetzungen gegenüber den Formulierungen des Standes der Technik nachgewiesen.
Folgende drei Zusammensetzungen wurden hergestellt:
Beispiel 1: (erfindungsgemäß) Antitranspirantspray mit verkapseltem Parfumöl und hautkühlendem Wirkstoff sowie unverkapseltem Parfumöl.
Vergleichsbeispiel A: Antitranspirantspray nur mit verkapseltem und unverkapseltem Parfumöl (ohne hautkühlenden Wirkstoff).
Vergleichsbeispiel B: Antitranspirantspray mit freiem Parfumöl (ohne verkapselte Komponenten).

| | **Beispiel 1 erfindungsgemäß** | **Vergleichsbeispiel A** | **Vergleichsbeispiel B** |
|---|---|---|---|
| Aluminiumchlorhydrat | 5% | 5% | 5% |
| Bentone Gel | 1% | 1% | 1% |
| Cyclopentasiloxan (Dow Corning 345) | 8% | 8% | 9% |
| Parfumöl 2, nicht verkapselt | 0,75% | 0,75% | 1% |
| Kapseln mit Parfumöl 1 und Menthyllactat nach Herstellbeispiel Nr. 5 | 1,25% | --- | --- |
| Kapseln mit Parfumöl 1 nach Herstellbeispiel 6 ohne Menthyllactat | --- | 1,25% | --- |
| Isobutan | 84% | 84% | 84% |

Die Formulierungen wurden an jeweils 20 Probanden über einen Zeitraum von 2 Wochen getestet. Die Probanden mussten die Produktleistung anhand der in der nachfolgenden Tabelle aufgelisteten Beurteilungskriterien bewerten. Die Bewertungsskala reicht von 1 = nicht zutreffend bis 7 = voll zutreffend. Die hierbei erzielten Ergebnisse belegen, dass die erfindungsgemäßen Zusammensetzungen gegenüber den Formulierungen des Standes der Technik eine verbesserte Produktleistung erbringen (siehe nachstehende Tabelle).

| **Beurteilungskriterium** | **Beispiel 1** | **Vergleichsbeispiel A** | **Vergleichsbeispiel B** |
|---|---|---|---|
| erzeugt einen angenehmen, kühlenden Effekt, besonders während sportlichen oder anderen körperlichen Aktivitäten | 6,0 | 5,5 | 5,5 |
| bewahrt einen angenehmen Duft, besonders während sportlichen oder anderen körperlichen Aktivitäten | 5,4 | 5,0 | 5,0 |
| schützt den ganzen Tag vor unangenehmem Körpergeruch | 5,9 | 5,6 | 5,5 |
| hat während des ganzen Tages einen erfrischenden Effekt | 5,8 | 5,2 | 4,9 |

Herstellungsbeispiele für die Verkapselung des Parfumöls und des hautkühlenden Wirkstoffs (alle Mengenangaben in Gew.%):

| | | Nr. 1 | Nr. 2 | Nr. 3 | Nr. 4 | Nr. 5 | Nr. 6 |
|---|---|---|---|---|---|---|---|
| Phase I | Wasser | 39,0 | 39,0 | 48,9 | 49,3 | 50,3 | 62,3 |
| | Glucidex 21 (Maltodextrin DE 20-35) | 36,0 | -- | -- | -- | -- | -- |
| | Nadex (Maltodextrin DE 9-12) | 4,0 | -- | -- | -- | -- | -- |
| | modifizierte Maisstärke | -- | 40,5 | -- | -- | -- | -- |
| | Natriumalginat | 0,8 | 0,3 | -- | -- | -- | -- |
| | Tylose H 10 | -- | -- | 20,5 | 28,0 | 24,0 | 24,0 |
| | Hydroxyethyl-cellulose | -- | -- | 9,5 | 2,0 | 5,0 | 5,0 |
| | Carragheenan | -- | -- | 0,8 | 0,5 | 0,5 | 0,5 |
| Phase II | Tween 20 | 0,2 | 0,2 | -- | -- | -- | -- |
| | Trilaureth-4-phosphate | -- | -- | 0,3 | 0,2 | 0,2 | 0,2 |
| | Parfum 1 | 10,0 | 10,0 | 10,0 | 5,0 | 8,0 | 8,0 |
| | Menthyllactat | 10,0 | -- | -- | -- | 12,0 | -- |
| | Isopulegol | -- | 10,0 | -- | -- | -- | -- |
| | Menthylpyrro- | -- | -- | 10,0 | -- | -- | -- |
| | lidoncarbonsäure | | | | | | |
| | Menthoxy-propandiol | -- | -- | -- | 15,0 | -- | -- |

Nach vorherigem getrennten Mischen wurde die Phase II zur Phase I gegeben und mit einem Schnellrührer homogenisiert. Anschließend wurde die Mischung sprühgetrocknet. Mit dem erhaltenen feinen Pulver konnten die erfindungsgemäßen Zusammensetzungen hergestellt werden.

| **Weitere erfindungsgemäße Formulierungsbeispiele: Antitranspirant-Sprays (Angaben in Gew.-%)** | | | | | | |
|---|---|---|---|---|---|---|
| | **2.1** | **2.2** | **2.3** | **2.4** | **2.5** | **2.6** |
| Dow Corning 245 | 10,0 | 10,0 | - | - | - | - |
| Dow Corning 246 | - | - | 10,0 | 10,0 | - | - |
| Dow Corning 345 | - | - | - | - | 10,0 | 10,0 |
| Isopropylmyristat | 5,0 | 5,0 | 5,0 | 5,0 | 5,0 | 5,0 |
| Tocopherylacetat | 0,2 | 0,2 | 0,2 | 0,2 | 0,2 | 0,2 |
| Triethylcitrat | - | - | - | - | - | 0,2 |
| Aluminiumchlorohydrat-Pulver | 5,0 | 5,0 | 5,0 | 5,0 | 5,0 | 5,0 |
| Aerosil® R 972 | 2,0 | - | 2,0 | 2,0 | 2,0 | - |
| Bentone Gel | - | 2,0 | - | - | - | 2,0 |
| Kapseln gemäß Herstellbeispiel 1 | 1,0 | - | - | - | - | - |
| Kapseln gemäß Herstellbeispiel 2 | - | 1,0 | - | - | - | - |
| Kapseln gemäß Herstellbeispiel 3 | - | - | 1,0 | - | - | - |
| Kapseln gemäß Herstellbeispiel 4 | - | - | - | 1,0 | - | - |
| Kapseln gemäß Herstellbeispiel 5 | - | - | - | - | 1,0 | - |
| ù Kapseln gemäß Herstellbeispiel 5 | - | - | - | - | - | 1,0 |
| n-Butan | ad 100 | ad 100 | ad 100 | ad 100 | ad 100 | ad 100 |

## Patentansprüche

1. Desodorierende Aerosolzusammensetzung, enthaltend
a) mindestens ein nicht-verkapseltes Parfumöl bzw. eine nicht-verkapselte Parfumöl-Mischung,
b) mindestens ein verkapseltes Parfumöl bzw. eine verkapselte Parfumöl-Mischung,
c) mindestens einen verkapselten hautkühlenden Wirkstoff und
d) mindestens ein Treibgas,
e) 0 - 2 Gew.% Wasser,
wobei die Parfumöle b) und der hautkühlende Wirkstoff c) mit mindestens einem wasserlöslichen Polymer verkapselt sind und die Zusammensetzung frei ist von ein - oder zweiwertigen C₁-C₆-Alkoholen, die keine Duftalkohole sind.

2. Zusammensetzung gemäß Anspruch 1, **dadurch gekennzeichnet, dass** das wasserlösliche Polymer ausgewählt ist aus Stärke, physikalisch und/oder chemisch modifizierter Stärke, Cellulosederivaten, Carragheenen, Alginaten, Pflanzengummen, Pektinen, Xanthanen, Dextrinen, Maltodextrinen, Polyvinylacetat, Polyvinylalkohol, Polyvinylpyrrolidin, Polyamiden, Polyestern und aus den Homo- und Copolymeren von Monomeren, ausgewählt aus Acrylsäure, Methacrylsäure, Maleinsäure, Fumarsäure, Itaconsäure sowie den Estern und den Salzen dieser Säuren, sowie beliebigen Mischungen dieser Polymeren.

3. Zusammensetzung gemäß einem der vorstehenden Ansprüche, **dadurch gekennzeichnet, dass** das wasserlösliche Polymer ausgewählt ist aus chemisch modifizierten Stärken, Carboxymethylcellulose, Hydroxyethylcellulose, Methylcellulose, Hydroxypropylmethylcellulose, Carragheenen und Alginaten, sowie beliebigen Mischungen dieser Polymeren.

4. Zusammensetzung gemäß einem der vorstehenden Ansprüche, **dadurch gekennzeichnet, dass** der hautkühlende Wirkstoff ausgewählt ist aus Menthol, Isopulegol, Menthylactat, Menthylpyrrolidoncarbonsäure, Menthylmethylether, Menthoxypropandiol, Menthonglycerinacetal (9-Methyl-6-(1-methylethyl)-1,4-dioxaspiro(4.5)decan-2-methanol), Monomenthylsuccinat und 2-Hydroxymethyl-3,5,5-trimethylcyclohexanol.

5. Zusammensetzung gemäß einem der vorstehenden Ansprüche, **dadurch gekennzeichnet, dass** das nicht-verkapselte Parfumöl a) bzw. die nicht-verkapselte Parfumöl-Mischung a) und das verkapselte Parfumöl b) bzw. die verkapselte Parfumöl-Mischung b) voneinander verschieden sind.

6. Zusammensetzung gemäß einem der vorstehenden Ansprüche, **dadurch gekennzeichnet, dass** sie frei von Ethanol oder Isopropanol ist.

7. Zusammensetzung gemäß einem der vorstehenden Ansprüche, **dadurch gekennzeichnet, dass** weiterhin mindestens ein desodorierender und/oder schweißhemmender Wirkstoff enthalten ist.

8. Zusammensetzung gemäß einem der vorstehenden Ansprüche, **dadurch gekennzeichnet, dass** weiterhin mindestens ein Verdickungsmittel, ausgewählt aus hydrophobierten Tonmineralien und pyrogenen Kieselsäuren, enthalten ist.
